# EUROPÄISCHE PATENTSCHRIFT

(11) **EP 1 181 941 B1**
(45) Veröffentlichungstag und Bekanntmachung des Hinweises auf die Patenterteilung: **05.04.2006**
(21) Anmeldenummer: 01120215.7
(22) Anmeldetag: 23.08.2001
(51) Int. Cl.: A61L 15/26, A61L 15/64, A61L 26/00

(54) **Beschichtungsmaterial für die Medizin aus resorbierbarem Material, Verfahren zu seiner Herstellung und Bereitstellung für die Medizin**
Absorbable medical coating material and method for its manufacture
Matériau de revêtement résorbable pour la médecine et son procédé de préparation

(30) Priorität: 24.08.2000 DE 10041684
(43) Veröffentlichungstag der Anmeldung: 27.02.2002
(73) Patentinhaber: PolyMedics Innovations GmbH, 70794 Filderstadt (DE)
(72) Erfinder: Hierlemann, Helmut, Dr., 73033 Göppingen (DE); Planck, Heinrich, Prof. Dr., 72622 Nürtingen (DE)
(74) Vertreter: Patentanwälte Ruff, Wilhelm, Beier, Dauster & Partner

(56) Entgegenhaltungen:
- EP-A- 0 401 844
- EP-A- 0 460 439
- EP-A- 0 600 237
- EP-A- 0 747 072
- EP-A- 0 835 894
- EP-A- 0 860 171
- EP-A- 0 908 482
- WO-A-94/11441
- WO-A-98/00065
- US-A- 5 475 063

## Beschreibung

Die vorliegende Erfindung betrifft Beschichtungsmaterial für die Medizin, insbesondere Wundkontaktmaterial aus resorbierbarem synthetischem Material.

Bei einer medizinischen Behandlung stellt sich häufig das Problem der Abschirmung von Körpergeweben gegen unerwünschte Einflüsse. Dies umfasst beispielsweise Abdeckung der Haut zum Schutz vor äusseren Einwirkungen oder bei Wunden wie im Falle von Verletzungen, Verschluss von Wunden bei chirurgischen Eingriffen, medizintechnische lmplantate sowie Trennung von Körpergewebe in der Adhäsionsprophylaxe, um unerwünschte Verwachsungen und Narbenbildung zu vermeiden.

Zu diesem Zweck wurden bioverträgliche Materialien auf Basis natürlicher und synthetischer Stoffe entwickelt. Bekannt sind Produkte aus nichtresorbierbaren Materialien wie Silikon, PUR, PP, PET, PA und PTFE sowie teilweise oder vollständig resorbierbare Produkte auf Basis von Collagen, Hyaluronsäure, Polysacchariden, Cellulose und ihren Derivaten, Milchsäure oder Glycolsäure.

Bei den bekannten Materialien ergeben sich Nachteile wegen ihres unvollständigen oder sehr langsamen Abbauverhaltens im Körper des Patienten. Ferner wegen unerwünschter Anhaftungen an Körpergewebe, was beispielsweise bei Anwendungen auf der Haut zu Problemen beim Verbandwechsel führt. Dies hat beim Patienten verlängerten Krankenstand zur Folge, verbunden auch mit persönlichen Unannehmlichkeiten wie Schmerzen und Mobilitätseinschränkungen.

Aus der EP 0 835 894 A2 ist ein Triblockpolymer aus Glykolid, Trimethylencarbonat und ε-Caprolacton bekannt, wobei das Hartsegment aus Glykolid und das Weichsegment aus Glykolid, Trimethylencarbonat und ε-Caprolacton bestehen. Das Triblockpolymer ist unter anderem zur Herstellung von Folien und textilen Flächengebilden geeignet.

In der EP 0 747 072 A2 ist ein Blockcopolymer beschrieben, das in Mischung mit einem oberflächenaktiven Mittel als formbares Knochenwachs verwendbar ist. Das Blockpolymer besteht aus einer größeren Menge an ε-Caprolacton und kleineren Mengen an mindestens einen weiteren copolymerisierbaren Monomer, unter anderem auch Lactid. In der Regel ist das Blockpolymer ein Sternpolymer, bei dem die Monomere an einen polyfunktionellen Initiator anpolymerisiert werden.

In der EP 0 860 171 A2 wird ein statistisches Terpolymer aus Trimethylencarbonat, ε-Caprolacton und Glykolid beschrieben, das ebenfalls als Knochenwachs verwendbar ist. Der Glykolidanteil beträgt maximal 60 Gew.-% und in der Regel nicht mehr als 40 Gew.-%. Dementspsrechend hoch sind die Anteile an Trimethylencarbonat und ε-Caprolacton.

Die Erfindung stellt sich die Aufgabe, ein bioverträgliches Beschichtungsmaterial aus resorbierbarem synthetischem Polymer zur Verfügung zu stellen, das die Probleme aus dem Stand der Technik überwindet, ein gutes Abbau- und Resorptionsverhalten in vivo in Kombination mit guten physikalischen und mechanischen Eigenschaften besitzt, das einfach und kostengünstig herzustellen sowie einfach und zuverlässig in der Medizin anzuwenden ist.

Diese Aufgabe wird gelöst durch ein Beschichtungsmaterial für die Medizin, insbesondere ein Wundkontaktmaterial, aus resorbierbarem synthetischem Material, dadurch gekennzeichnet, dass es aus einem Terpolymer auf Basis von Lactid, Trimethylencarbonat und ε-Caprolacton mit einem Gehalt an Lactid von 65 bis höchstens 85 Gew.-%, insbesondere höchstens 80 Gew.-%, Trimethylencarbonat im Bereich von 5 bis 20 Gew.-%, insbesondere 10 bis 20 Gew.-%, und ε-Caprolacton im Bereich von 5 bis 20 Gew.%, insbesondere 5 bis 15 Gew.-%, gebildet ist, wobei sein Abbauverhalten durch die Anteilsverhältnisse der Monomeren, insbesondere den Lactidanteil, bestimmt ist und wobei der Gesamtmonomerengehalt im Terpolymer bei bis zu 10 Gew.-% liegt.

In einer bevorzugten Ausführungsform können im Terpolymer die Monomere Lactid/Trimethylencarbonat/ε-Caprolacton in Verhältnissen im Bereich von 85/10/5 bis 70/20/10 Gew.-% vorliegen. Das erfindungsgemässe Terpolymer besitzt einen Gesamtmonomergehalt von bis zu 10 Gew.-% der Monomere Lactid, Trimethylencarbonat und ε-Caprolacton. Vorzugsweise liegt der Monomergehalt bei 1 bis 9 Gew.-%.

Als Lactidkomponenten können im Terpolymer L-Lactid, DL-Lactid und/oder meso-Lactid verwendet sein. Besonders bevorzugt kann die Lactidkomponente DL-Lactid sein.

Das erfindungsgemässe Beschichtungsmaterial kann sich dadurch auszeichnen, dass es im Temperaturbereich von 20 bis 40 °C, insbesondere im Bereich von 28 bis 37 °C einen Glasumwandlungspunkt aufweist. Hiermit ergeben sich besondere Vorteile für den Einsatz des Materials beim Patienten, dessen Körpertemperatur in diesem Bereich liegt.

Das Terpolymer kann ab initio ein Molekulargewicht im Bereich von 80000 Dalton bis 400000 Dalton, insbesondere von 90000 bis 250000 Dalton, aufweisen. Nach einer etwaigen Gammastrahlenbehandlung, beispielsweise von 25 kGy wie zum Zwecke der Sterilisierung angewendet, kann das Terpolymer ein Molekulargewicht im Bereich von 50000 bis 150000 Dalton, insbesondere von 60000 bis 90000 Dalton, aufweisen. Die Gammastrahlenbehandlung des erfindungsgemässen Materials wird unten noch ausführlicher erläutert.

Weiterhin kann das Terpolymer ab initio eine inhärente Viskosität von 0,8 bis 2,5 dl/g, insbesondere von 1,0 bis 2,0 dl/g, aufweisen. Nach einer etwaigen Gammastrahlenbehandlung in sterilisiertem Zustand kann das Terpolymer eine inhärente Viskosität von 0,7 bis 1,2 dl/g, insbesondere 0,7 bis 1,0 dl/g, aufweisen.

Das erfindungsgemässe Material zeichnet sich besonders durch vorteilhafte mechanische Eigenschaften und gute Handhabbarkeit aus. Das Material kann eine Dehnung von mindestens 50 % bei 37 °C aufweisen. Sein Modul kann bei Körpertemperatur weniger als 1000 N/mm² betragen. Seine Reissdehnung kann bei 37 °C im Bereich von 50 bis 400 %, insbesondere 100 bis 300 % liegen. Die Reissfestigkeit des erfindungsgemässen Materials kann weniger als 30 N/mm² betragen.

Bei Temperaturen im Bereich der Raumtemperatur und darunter ist das Material gemäss der Erfindung gut handhabbar, flexibel und dehnbar. Auf diese Weise kann es für medizinische Anwendung vorbereitet, beispielsweise auf eine gewünschte Grösse zurechtgeschnitten, und beim Patienten aufgebracht oder eingesetzt werden. Nach Kontakt mit dem Körper des Patienten erwärmt sich das Material zunehmend, bis auf dessen Körpertemperatur. Dabei wird das erfindungsgemässe Material zunehmend plastisch verformbar, weicher, flexibler und dehnbarer, bis es fast einen fliessfähigen Zustand erreicht. Auf diese Weise ist eine optimale Anpassung an das zu behandelnde Körpergebiet des Patienten möglich.

Bei Temperaturen im Bereich der Körpertemperatur des Menschen von 37 °C kann die Dehnbarkeit des Materials bis zu 250 % betragen. Bevorzugt kann seine Dehnbarkeit bei 37 °C bei 100 % liegen. Die zunehmende Flexibilisierung des Polymermaterials wird auch durch Wasseraufnahme positiv beeinflusst.

Das erfindungsgemässe Material kann sich vorteilhaft durch Wasserdampfdurchlässigkeit auszeichnen. Bei drucklosem Wasserdurchtritt kann seine Wasserdampfdurchlässigkeit 20 bis 100 ml/m²/h, insbesondere 40 bis 80 ml/m²/h, betragen. Auf diese Weise kann sowohl ein Flüssigkeitsstau als auch eine Austrocknung im Wundbereich vermieden werden. Ein gemäss der Erfindung ausgebildetes medizintechnisches Produkt kann in physiologischer Weise mit dem behandelten Körperbereich des Patienten interagieren. Ferner ist Stoffwechselaktivität im behandelten Körperbereich möglich. Dies stimuliert Wundheilungsvorgänge und unterstützt lokale Abwehrmechanismen. Das erfindungemässe Beschichtungsmaterial kann sich vorteilhaft durch Barrierefunktion gegenüber Mikroorganismen auszeichnen. Auf diese Weise kann eine Infektion verhindert werden, was für die Heilung in jedem Fall günstig ist. Bei topischer Anwendung ist ausserdem eine Temperaturregulation des Wundbereichs möglich, was ebenfalls die Heilung begünstigt.

Besonders vorteilhaft kann sich das erfindungsgemässe Terpolymer dadurch auszeichnen, dass es im Körper eines Patienten vollständig resorbierbar ist. Dies ist für die gute Körperverträglichkeit beim Einsatz in Mensch und Tier besonders günstig. Der Abbau des erfindungsgemässen Polymers erfolgt im Körper eines Tieres oder eines Menschen durch Stoffwechselvorgänge. An der Umsetzung sind Körper- und Gewebeflüssigkeiten beteiligt. Durch Hydrolyse wird die Polymerkette in kleinere und leichter lösliche Fragmente gespalten. Die Bruchstücke werden ggf. unter Beteiligung enzymatischer Prozesse weiter abgebaut. Die Abbauprodukte werden durch das Stoffwechselsystem abtransportiert und wie andere Stoffwechselschlacken aus dem Organismus ausgeschieden. Für eine gute Verträglichkeit des resorbierbaren Nahtmaterials beim Patienten ist es wichtig, dass sich während des Abbauvorganges keine schädlichen Metaboliten bilden oder anreichern. Polylactid zeichnet sich insbesondere dadurch aus, dass bei seiner Zerset-zung in vivo keine toxischen Zerfallsprodukte gebildet werden. Die weitere Monomere im erfindungsgemässen Terpolymer verwendeten Trimethylencarbonat (TMC) und Caprolacton sind ebenfalls durch gute Verträglichkeit und Vermeidung toxischer Reaktionen gekennzeichnet.

Vorteilhafte physiologische Eigenschaften weist das erfindungsgemässe Material ferner dadurch auf, dass dem Terpolymer kein Weichmacher zugesetzt ist. Seine Weichheit und Flexibilität sind durch die molekulare Zusammensetzung und Polymerstruktur bedingt. Insbesondere eine höherer Gehalt an Caprolacton und TMC fördern die Weichheit des Terpolymers. Ein hoher Gehalt an Lactid begünstigt Härte und Steifheit des Polymers.

Das Material gemäss der Erfindung kann sich dadurch auszeichnen, dass seine Degradationszeit in vivo 15 bis 60 Tage, insbesondere 20 bis 45 Tage, bevorzugt 25 bis 35 Tage beträgt. Seine Resorptionszeit in vivo kann 70 bis 120 Tage, insbesondere 80 bis 100 Tage betragen.

Während des Abbaus des erfindungsgemässen Materials ergibt sich mit Vorteil ein leicht saures Milieu von etwa pH 5. Dies ist ein physiologisch günstiger pH-Bereich, der auch den Bedingungen des menschlichen Körpers entspricht. Insbesondere die Hautoberfläche des Menschen weist einen pH-Wert im Bereich von pH 5 auf. Diese pH-Bedingungen wirken bakterizid und wundstimulierend, was für die Heilung vorteilhaft ist.

Das Material gemäss der Erfindung zeichnet sich dadurch aus, dass sein Abbauverhalten durch die Anteilsverhältnisse der Monomeren, insbesondere den Lactidanteil bestimmt ist. Das Degradationsverhalten des erfindungsgemässen Terpolymers kann durch Variation des Lactidanteils im Polymer beeinflusst, bevorzugt eingestellt werden. Ein anderer Einflussfaktor, durch dessen Variation das Abbauverhalten des erfindungsgemässen Polymers verändert werden kann, ist die Intensität und Dauer einer Gammabestrahlung. Die Behandlung mit Gammastrahlen kann mit einem teilweisen Molekulargewichtsabbau verbunden sein, der sich in verkürzten Abbauzeiten äussert. Auf diese Weise ist es möglich, die Eigenschaften des Terpolymers gemäss der Erfindung nach den für den Anwendungsfall vorteilhaften Erfordernissen anzupassen. In einer möglichen Ausführungsform der Erfindung kann eine mit Hilfe von Gammastrahlen durchgeführte Sterilisation gleichzeitig zur Steuerung des Degradationsverhaltens von aus dem erfindungsgemässen Terpolymer hergestellten medizintechnischen Produkten dienen.

Wie sich durch die Degradation des erfindungsgemässen Terpolymers im Laufe der Zeit auch seine physikalischen und mechanischen Eigenschaften verändern, ist in der beigefügten Fig. 1 dargestellt. Eine Membran hergestellt mit DL-Lactid von 80 bis 120 µm Dicke und bei 25 kGy sterilisiert wird bei 37 °C und pH 7,4 in Sörensenpuffer abgebaut. In der Figur ist die prozentuale Abnahme der inhärenten Viskosität, der maximalen Belastung und der Reissdehnung aufgetragen. Innerhalb von sechs Wochen zeigt sich eine Abnahme der Werte auf 20 bis 40 % der Ausgangswerte.

Ferner können dem erfindungsgemässen Material mit Vorteil eine oder mehrere medizinisch wirksame Substanzen zugesetzt sein. Solche dotierten Wirksubstanzen können beispielsweise Medikamente, Impfstoffe, Antibiotika, Antiseptika, Wachstumsfaktoren und dergleichen sein.

Zur Anwendung in der Medizin kann das Material gemäss der Erfindung in Form eines ein- oder mehrschichtigen Schichtmaterials, insbesondere in Form einer Folie vorliegen. In einer Ausführungsform kann das Material gemäss der Erfindung in Form einer porösen Membran vorliegen. In einer weiteren Ausführungsform kann das Material gemäss der Erfindung in Form eines Formkörpers vorliegen. In einer anderen weiteren Ausführungsform kann das Material in Form eines Netzes vorliegen. In noch einer anderen Ausführungsform kann das Material gemäss der Erfindung in Form einer Hohlmembran vorliegen. In einer besonderen Ausführungsform kann das Material gemäss der Erfindung in Form einer Lösung vorliegen. In noch einer weiteren Ausführungsform kann das Material gemäss der Erfindung in Form eines Vlieses vorliegen. Ein solches Vlies kann insbesondere nach einer Melt-Blow-Technik als Melt-Blown-Vlies ausgebildet sein. In einer besonderen Ausführungsform kann das Material gemäss der Erfindung in Form eines Kompositverbundes mit anderen biokompatiblen Strukturen kombiniert vorliegen. Solche Verbundkomponenten können aus resorbierbarem und/oder nicht resorbierbarem Material gebildet sein. Die Verbundkomponenten können als textile Strukturen vorgesehen sein. Eine Verbundbildung kann nach textilen und/oder thermoplastischen Verfahrensweisen vorgenommen sein.

Mit Vorteil kann sich das Material gemäss der Erfindung dadurch auszeichnen, dass es eine poröse Struktur aufweist. Insbesondere kann seine Porosität mehr als 85 % betragen. Dabei kann die Porengrösse mehr als 1 µm, insbesondere mehr als 10 µm, betragen.

In einer besonderen Ausführungsform kann sich das erfindungsgemässe Material als Carrier oder Matrix für Zellkulturen durch gute Proliferationseigenschaften auszeichnen.

In welcher Form das erfindungsgemässe Material ausgebildet ist, hängt insbesondere von der vorgesehenen Anwendung ab. Zur topischen Anwendung auf der Haut des Patienten sind insbesondere Flächengebilde wie Filme, Folien, Netze oder Membranen geeignet. Auch flüssige Ausführungsformen können für äusserliche Anwendungen vorteilhaft sein, wie beispielsweise Sprühverbände.

Zur Anwendung bei chirurgischen Eingriffen im Körperinneren des Patienten können ebenfalls Flächengebilde, aber mit Vorteil auch Formkörper, Hohlmembranen, Filamente oder flüssige Formulierungen sowie Beschichtungen von Implantaten vorgesehen sein. Als Beispiele für erfindungsgemässe Flächengebilde sind Filme, Folien, Membranen, Vliese, textile Flächengebilde wie Gewirke, Gewebe, Gestricke und dergleichen sowie Verbundstoffe wie Laminate zu nennen.

Die Erfindung umfasst ferner ein Verfahren zur Herstellung eines Beschichtungsmaterials für die Medizin, insbesondere eines Wundkontaktmaterials aus resorbierbarem synthetischem Material, dadurch gekennzeichnet, dass auf Basis von Lactid, Trimethylencarbonat und ε-Caprolacton als Monomere durch Polymerisation ein Terpolymer mit einem Gehalt an Lactid von 65 bis höchstens 85 Gew.-%, Trimethylencarbonat im Bereich von 5 bis 20 Gew.-% und ε-Caprolacton im Bereich von 5 bis 20 Gew.-% gebildet und zu einem Produkt für medizinische Anwendung ausgebildet wird, wobei sein Abbauverhalten durch die Anteilsverhältnisse der Monomeren, insbesondere den Lactidanteil, eingestellt wird und wobei der Gesamtmonomerengehalt im Terpolymer bis zu 10 Gew.-% betragen kann.

In Weiterführung des Herstellungsverfahrens kann das Terpolymer in einem geeigneten Lösemittel gelöst und durch Ausstreichtechniken zu Flächengebilden, wie Folien oder Membranen, ausgebildet werden. Als geeignete Lösemittel sind beispielsweise Ethylacetat, Aceton, Chloroform, Methylenchlorid, Tetrachlorkohlenstoff, Dimethylacetamid, Formamid, Tetrahydrofuran, Dioxan, Dimethylsulfoxid sowie deren Mischungen zu nennen.

In einer anderen Ausführungsform des Herstellungsverfahrens kann das Terpolymer in einem geeigneten Lösemittel gelöst und durch Phaseninversionstechniken zu einer porösen Struktur ausgebildet werden. Hierbei wird mittels zweier unterschiedlicher miteinander mischbarer Lösemittel, deren eines das Polymer löst und das andere nicht, das Polymer so ausgefällt, dass eine poröse Membran zurückbleibt. Eine weitere Methode, eine poröse Membran herzustellen, kann beispielsweise durch Gefriertrocknung erfolgen, wobei das Polymer ausfällt und das gefrorene Lösemittel entfernt wird, so dass anstelle des Lösemittels Poren zurückbleiben. Eine andere Art eine poröse Membran herzustellen, ist die Sprühvliestechnik. Durch geeignete Wahl der Verfahrensbedingungen kann die Porenstruktur und Porengrösse in der Membran nach Wunsch ausgebildet werden.

Bei einer weiteren Verfahrensweise kann das erfindungsgemässe Terpolymer thermoplastisch durch Extrusion verarbeitet werden. Auf diese Weise können mittels Extruder und Spinndüsen Filamente, Monofilamente oder Multifilamente ausgebildet werden. Solche Filamente können nach bekannten Techniken zu medizintechnischen Produkten gemäss der Erfindung verarbeitet werden. Ferner können mittels Extruder Blasfolien, Pressfolien oder Walzfolien ausgebildet werden. Ausserdem können mittels Extruder nach der Melt-Blow-Technik poröse Strukturen beliebiger Form ausgebildet werden. Für die Ausbildung von Formkörpern und freien Strukturen kann mit Vorteil die Stapelfasertechnologie angewendet werden.

Die Verfahrensweisen zur Ausbildung bestimmter Formen des erfindungsgemässen Materials sind den Fachleuten bekannt und sollen daher nicht ausführlicher besprochen werden.

Je nach Ausführungsform kann das erfindungsgemässe Beschichtungsmaterial in unterschiedlichen Dicken vorliegen. Eine erfindungsgemässe Membran kann eine Dicke im Bereich von 20 bis 500 µm, insbesondere 50 bis 250 µm, bevorzugt 100 bis 150 µm aufweisen. Eine erfindungsgemässe Folie kann eine Dicke im Bereich von 10 bis 100 µm, insbesondere 20 bis 50 µm, bevorzugt 30 bis 40 µm aufweisen.

Ein gemäss der vorliegenden Erfindung ausgebildetes Material eignet sich mit Vorteil zur Verwendung als Wundabdeckungsmaterial bei der medizinischen Behandlung von Mensch oder Tier.

In einer bevorzugten Ausführungsform eignet es sich zur Verwendung als Hautersatzmaterial bei der medizinischen Behandlung von Mensch oder Tier.

In einer anderen Ausführungsform eignet es sich zur Verwendung zur Adhäsionsprophylaxe bei der medizinischen Behandlung von Mensch oder Tier.

In einer besonderen Ausführungsform eignet es sich zur Verwendung als Matrix für Zellkulturen.

In einer weiteren Ausführungsform eignet es sich zur Verwendung zur Beschichtung von medizintechnischen Implantaten für die medizinische Behandlung von Mensch oder Tier.

Mit besonderem Vorteil kann das erfindungsgemässe Material bei der medizinischen Behandlung der Haut verwendet werden. Diese Anwendungen umfassen beispielsweise Wundbehandlung, Hautersatz oder auch Schutz gesunder Haut vor dem Einwirken schädlicher Umgebungseinflüsse. Unter Wundbehandlung sind Wunden ausgelöst durch Trauma wie beispielsweise Schürfwunden, chronische Wunden wie beispielsweise Hautläsion bei Diabetes mellitus, venös bedingtes Ulcus, Dekubitusulcus, arterielle Verschlusskrankenheiten und auch Verbrennungen zu verstehen.

Insbesondere Verbrennungswunden sowie tiefdermale Wunden zerstören die Schutzfunktion der Haut und stellen ein Risiko für das Überleben des Patienten dar. Besonders bei zweit- und drittgradigen Verbrennungswunden sowie bei Spalthautentnahmestellen zur Transplantation ist ein hoher Wasserverlust, Temperaturabfall, eine Zunahme der Keimdurchlässigkeit verbunden mit erhöhtem Infektionsrisiko zu beobachten. Bedarf an synthetischem Verbandsmaterial und/oder an Hautersatzmaterial besteht in der Verbrennungsmedizin, der plastischen Chirurgie, auch im Bereich Mund, Kiefer und Gesicht einschliesslich der Parodontologie und Kieferorthopädie, in der Handchirurgie und Wiederherstellungschirurgie sowie auch in der Unfallchirurgie.

Beim Hautersatz können verschiedene Ersatzstrategien verfolgt werden. Beim epidermalen Ersatzverfahren wird eine Hautabdeckung mit autologen Keratinazyten bevorzugt in Verbindung mit einer Transportmatrix wie beispielsweise einem inerten Polymerfilm aus resorbierbarem Material vorgenommen. Beim dermalen Ersatzverfahren wird eine synthetische dermale Matrix für die Infiltration und Reorganisation körpereigener Zellen zur Verfügung gestellt. Beim Komposithautersatzverfahren wird eine Kombination aus dermalem Matrixersatz mit epidermalen und/oder dermisständigen Zellen angewendet.

Sowohl für einen optimalen Wundverband wie für einen Hautersatz sind an ein synthetisches Polymermaterial Anforderungen gestellt, sich an den Eigenschaften menschlicher oder tierischer Haut zu orientieren und ausserdem mit dem Wundbett in physiologischer Weise zu interagieren. Das erfindungsgemässe Terpolymermaterial erfüllt die gestellten Anforderungen. Es zeichnet sich durch eine gute Adhärenz an das Wundbett aus. Es zeigt eine Barrierefunktion gegen Mikroorganismen. Ferner ist es ausreichend wasserdampfdurchlässig und ermöglicht eine Temperaturregulation über den Verband. Ausserdem werden Wundheilungsmechanismen angeregt und lokale Abwehrmechanismen unterstützt. Das physiologisch gut verträgliche resorbierbare Produkt erlaubt eine weitgehend entzündungsfreie Integration in die Empfängerstruktur mit kontrollierten Abbau und Umbau (Remodeling) des behandelten Körpergewebes. Im Falle der Verwendung als äusserer Wundverband kann die Häufigkeit des Verbandwechsels auf ein- bis zweimal pro Woche verringert werden, was zu einer deutlichen Schmerzverringerung beim Patienten, vermindertem Infektionsrisiko und signifikanter Abnahme des Wundtraumas führt, bei dem frisch epithelisierte Hautareale abgelöst werden. Auf diese Weise kann bei der Hautbehandlung Narbenbildung verhindert und neugebildete Haut mit langfristig guter mechanischer und ästhetischer Qualität erhalten werden.

Ferner kann das medizintechnische Material, wie es oben beschrieben ist, zur Prophylaxe von Adhäsionen bei chirurgischen Eingriffen in der Humanmedizin und Veterinärmedizin verwendet werden. Das erfindungsgemässe Material kann je nach den medizinischen Erfordernissen und der gewünschten Einsatzweise in verschiedenen Formen verwendet werden. Beispielsweise in Form einer Membran, einer Lösung oder eines Sprays. Die Anwendung des resorbierbaren Materials gemäss der Erfindung kann unerwünschte Verwachsungen von Körpergewebe verhindem und fördert die Endothelbildung.

Eine andere Bereitstellung des oben genannten Materials dient zur Verwendung für die Beschichtung von medizintechnischen Implantaten, die in den Körper des Patienten eingesetzt werden. Als Beispiel ist die Beschichtung von Stents mit einer Lösung des erfindungsgemässen Terpolymers zu nennen. Solche Stents können für tracheale, gastrointestinale, urethrale und vasculäre Anwendungen eingesetzt werden.

Wegen seiner günstigen Eigenschaften im Hinblick auf eine Infiltration von Zellen ist das erfindungsgemässe Material auch als Carrier oder Matrix für Zellkulturen im Rahmen des Tissue Engineering anwendbar. Eine solche Carrier/Matrix kann in Form einer Folie, Membran, eines Vlieses oder einer anderen textilen Struktur aus dem Terpolymer gemäss der Erfindung ausgebildet sein.

Das gemäss der Erfindung hergestellte resorbierbare Material kann zur Bereitstellung für medizinische Verwendung auf an sich bekannte Weise vorbereitet werden. Insbesondere kann das erfindungsgemässe Polymermaterial in geeigneter Weise sterilisiert werden. Ein zweckmässiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfasst die Behandlung mit Gammastrahlung. Ein anderes Verfahren zur Sterilisierung des erfindungsgemässen Polymermaterials für medizinische Zwecke umfasst die Verwendung von Ethylenoxid.

Mit Vorteil kann aus dem erfindungsgemässen Polymer hergestelltes medizinisches Material in zweckmässiger Grösse zugeschnitten gebrauchsfertig in geeigneter Weise verpackt vorliegen. Wegen der hydrolytischen Zersetzbarkeit des erfindungsgemässen Terpolymermaterials sind die medizinischen Produkte bei ihrer Lagerung vor Feuchtigkeit und erhöhten Temperaturen zu schützen, damit die Festigkeitseigenschaften bis zur Verwendung voll erhalten bleiben. Mit Vorteil können gemäss der Erfindung hergestellte medizinische Produkte in gebrauchsbereitem Zustand getrocknet und in geeigneter Weise verpackt werden. Zweckmässigerweise kann dies durch eine vor Feuchtigkeit schützende Verpackung geschehen, insbesondere einer Verpackung aus feuchtigkeitsundurchlässigem Folienmaterial, bevorzugt einer Vakuumverpackung. Ferner durch Auswahl eines trockenen kühlen Lagerortes.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiel 1

### Herstellung eines Polymers mit Lactid/Caprolacton/TMC im Verhältnis 75/10/15

Zur Herstellung eines Terpolymers werden 1500 g DL-Lactid, 200 g ε-Caprolacton und 300 g Trimethylencarbonat (TMC) unter Rühren vermischt. Nach Zugabe des Katalysators 0,4 g Zinnoctoat (entspr. 0,02 Gew.-%) wird unter Rühren auf 150 °C erhitzt und 24 Stunden bei dieser Temperatur weiterpolymerisiert. Zum Ablassen des Reaktionsgemisches wird die Temperatur auf 180 bis 200 °C erhöht, das Polymer ausgetragen und nach dem Erkalten auf eine Komgrösse von 5 mm gemahlen.

### Beispiel 2

### Herstellung eines Polymers mit Lactid/Caprolacton/TMC im Verhältnis 80/10/10

Zur Herstellung eines Terpolymers werden 1600 g DL-Lactid, 200 g ε zur Verwendung -Caprolacton und 200 g TMC unter Rühren vermischt. Nach Zugabe des Katalysators Zinnoctoat (0,4 g entspr. 0,02 Gew.-%) wird unter Rühren auf 170 °C erhitzt und 24 Stunden bei dieser Temperatur weiterpolymerisiert. Zum Ablassen des Reaktionsgemisches wird die Temperatur auf 180 bis 200 °C erhöht, das Polymer ausgetragen und nach dem Erkalten auf eine Korngrösse von 5 mm gemahlen.

### Beispiel 3

### Herstellung einer Folie aus Lösung

In einem Glasreaktor werden 240 g granuliertes Polymer mit 800 g Ethylacetat versetzt und unter Rühren eine ca. 23 %ige Polymerlösung hergestellt. Nach 4 Stunden Rühren wird die Polymerlösung unter Druck filtriert (44 µm Filter / mesh-No. 325 Stahldrahtgewebe) und 12 Stunden entgast. Die filtrierte und entgaste Polymerlösung wird auf eine Glasplatte gegossen und mit einer 250 µm Rakel ausgestrichen. Die mit der Polymerlösung beschichtete Glasplatte wird für 2 Stunden bei Raumtemperatur unter dem Abzug belassen, anschliessend 24 Stunden lang bei 50 °C zur Reduzierung des Restlösemittelgehalts nachgetrocknet. Die so hergestellten Folien haben eine Trockenschichtdicke von 20 ± 5 µm, eine Reissfestigkeit von > 10 N/mm², einen Modul von < 1000 N/mm² und eine Reissdehnung von > 50 %.

### Beispiel 4

### Herstellung einer Membran aus Lösung

Die Herstellung, Filtration und Entgasung der Polymerlösung erfolgt analog Beispiel 3. Anstelle von Ethylacetat wird als Lösungsmittel 1,4-Dioxan verwendet. Die filtrierte und entgaste Polymerlösung wird auf eine Glasplatte gegossen und mit einer 400 µm Rakel ausgestrichen. Die mit der Polymerlösung beschichtete Glasplatte wird sofort bei -10 °C zwei Stunden lang gekühlt und anschliessend bei einem Vakuum von > 0,5 mbar gefriergetrocknet. Der Gefriertrocknungsprozess ist nach 12 Stunden beendet. Die so hergestellten Membranen haben eine Schichtdicke von 80 bis 100 µm, eine Porosität von > 85 % bei Porengrössen von 5 bis 50 µm, eine Reissfestigkeit von > 2 N/mm², einen Modul von < 1000 N/mm² und eine Reissdehnung von > 50 %.

### Beispiel 5

### Herstellung eines Vlieses durch thermoplastische Verarbeitung

1000 g granuliertes Polymer nach Beispiel 1 werden unter Schutzgas (Argon, Stickstoff) in einem Extruder einer Spinndüse zugeführt und unmittelbar vor dem Austritt mit einem sehr schnellen Luftstrom extrudiert und verstreckt. Die Schmelztemperatur und die Temperatur des Luftstromes betragen 150 bis 170 °C, die Geschwindigkeit der Anblasung beträgt 5 bis 200 m/s. Die Polymerschmelze wird durch den hohen Luftdurchsatz in faserartige Teilchen umgewandelt, auf einer Krempel zu einem Flor feiner Fasern abgelegt. Das Rohvlies kann nun in Nachbearbeitungsschritten chemisch (mit Lösungsmitteln), thermisch (über heisse Walzen oder Kalander) oder mechanisch (Krempel, Vernadelungsmaschinen) verfestigt werden.

## Patentansprüche

1. Beschichtungsmaterial für die Medizin, insbesondere Wundkontaktmaterial, aus resorbierbarem synthetischen Material, **dadurch gekennzeichnet, dass** es aus einem Terpolymer auf Basis von Lactid, Trimethylencarbonat und ε-Caprolacton mit statistischer Verteilung der MOnomeren, mit einem Gehalt an Lactid von 65 bis höchstens 85 Gew.-%, Trimethylencarbonat im Bereich von 5 bis 20 Gew.-% und ε-Caprolacton im Bereich von 5 bis 20 Gew.-% gebildet ist, wobei sein Abbauverhalten durch die Anteilsverhältnisse der Monomeren, insbesondere den Lactidanteil bestimmt ist, und wobei der Gesamtmonomerengehalt im Terpolymer bei bis zu 10 Gew.-% liegt.

2. Material nach Anspruch 1, **dadurch gekennzeichnet, dass** im Terpolymer die Monomere Lactid/Trimethylencarbonat/ ε-Caprolacton in Verhältnissen im Bereich von 85/10/5 bis 70/20/10 Gew.-% vorliegen.

3. Material nach Anspruch 1 oder 2, **dadurch gekennzeichnet, dass** es einen Glasumwandlungspunkt im Bereich von 20 °C bis 40 °C, insbesondere im Bereich von 28 bis 37 °C aufweist.

4. Material nach einem der vorhergehenden Ansprüche, **dadurch gekennzeichnet, dass** es in sterilisiertem Zustand nach Behandlung mit Gammastrahlen ein Molekulargewicht von 50000 bis 150000 Dalton, insbesondere 60000 bis 90000 Dalton, aufweist.

5. Material nach einem der vorhergehenden Ansprüche, **dadurch gekennzeichnet, dass** es in sterilisiertem Zustand nach Behandlung mit Gammastrahlen (25 kGy) eine inhärente Viskosität von 0,7 bis 1,2 dl/g aufweist.

6. Material nach einem der vorhergehenden Ansprüche, **dadurch gekennzeichnet, dass** es eine Dehnung von mindestens 50 % bei 37 °C aufweist.

7. Material nach einem der vorhergehenden Ansprüche, **dadurch gekennzeichnet, dass** seine Degradationszeit in vivo 15 bis 60 Tage, insbesondere 20 bis 45 Tage, bevorzugt 25 bis 35 Tage beträgt.

8. Material nach einem der vorhergehenden Ansprüche, **dadurch gekennzeichnet, dass** seine Resorptionszeit in vivo 70 bis 120 Tage, insbesondere 80 bis 100 Tage beträgt.

9. Material nach einem der vorhergehenden Ansprüche, **dadurch gekennzeichnet, dass** es medizinisch wirksame Substanzen, insbesondere Medikamente enthält.

10. Material nach einem der vorhergehenden Ansprüche, **dadurch gekennzeichnet, dass** es als ein- oder mehrschichtiges Schichtmaterial vorliegt.

11. Material nach einem der vorhergehenden Ansprüche, **dadurch gekennzeichnet, dass** es in Form einer Folie vorliegt.

12. Material nach einem der Ansprüche 10 oder 11, **dadurch gekennzeichnet, dass** mindestens eine Schicht in Form einer porösen Membran vorliegt.

13. Material nach einem der Ansprüche 10 bis 12, **dadurch gekennzeichnet, dass** mindestens eine Schicht in Form eines Netzes vorliegt.

14. Material nach einem der Ansprüche 10 bis 12, **dadurch gekennzeichnet, dass** es mindestens eine Schicht in Form einer Hohlmembran vorliegt.

15. Material nach einem der Ansprüche 10 bis 12, **dadurch gekennzeichnet, dass** es in Form eines Vlieses, insbesondere eines Melt-Blown-Vlieses vorliegt.

16. Material nach einem der vorhergehenden Ansprüche, **dadurch gekennzeichnet, dass** es porös ist, seine Porosität insbesondere mehr als 85 % beträgt.

17. Material nach einem der vorhergehenden Ansprüche, **dadurch gekennzeichnet, dass** seine Porengröße mehr als 1 µm, insbesondere mehr als 10 µm, beträgt.

18. Material nach einem der vorhergehenden Ansprüche, **dadurch gekennzeichnet, dass** es in Form einer Lösung vorliegt.

19. Verfahren zur Herstellung eines Beschichtungsmaterials für die Medizin, insbesondere eines Wundkontaktmaterials aus resorbierbarem synthetischen Matieral, **dadurch gekennzeichnet, dass** durch statistische Polymerisation ein Terpolymer ausgebildet wird, auf Basis von Lactid, Trimethylencarbonat und ε-Caprolacton als Monomere, mit einem Gehalt an Lactid von 65 bis höchstens 85 Gew.-%, Trimethylencarbonat im Bereich von 5 bis 20 Gew.-% und ε-Caprolacton im Bereich von 5 bis 20 Gew.-%, wobei nach der Polymerisation ein Gesamtmonomergehalt von bis zu 10 Gew.-% verbleibt, und das Terpolymer nach Polymerverarbeitungsmethoden zu einem Produkt für medizinische Anwendung ausgebildet wird, wobei sein Abbauverhalten durch die Anteilsverhältnisse der Monomeren, insbesondere der Lactidanteil, eingestellt wird und wobei der Gesamtmonomergehalt im Terpolymer bis zu 10 Gew.-% betragen kann.

20. Verfahren nach Anspruch 19, **dadurch gekennzeichnet, dass** das Terpolymer in einem Lösemittel gelöst und durch Ausstreichtechniken zu Flächengebilden ausgebildet wird.

21. Verfahren nach Anspruch 19, **dadurch gekennzeichnet, dass** das Terpolymer thermoplastisch durch Extrusion zu Filamenten oder Folien ausgebildet wird.

22. Verfahren nach Anspruch 19, **dadurch gekennzeichnet, dass** das Terpolymer thermoplastisch durch Melt-Blow-Technik zu porösen Strukturen ausgebildet wird.

23. Verfahren nach Anspruch 19, **dadurch gekennzeichnet, dass** das Terpolymer in einem Lösemittel gelöst und durch Phaseninversionstechniken zu einer porösen Struktur ausgebildet wird.

24. Verwendung eines Beschichtungsmaterials nach einem der Ansprüche 1 bis 18 zur Herstellung eines Wundabdeckungsmaterials für die medizinische Behandlung von Mensch und Tier.

25. Verwendung eines Beschichtungsmaterials nach einem der Ansprüche 1 bis 18 zur Herstellung eines Hautersatzmaterials für die medizinische Behandlung von Mensch und Tier.

26. Verwendung eines Beschichtungsmaterials nach einem der Ansprüche 1 bis 18 zur Herstellung eines Adhäsionsprophylaxematerials für die medizinische Behandlung von Mensch und Tier.

27. Verwendung eines Beschichtungsmaterials nach einem der Ansprüche 1 bis 18 als Matrix für Zellkulturen.

28. Verwendung eines Beschichtungsmaterials nach! einem der Ansprüche 1 bis 18 als Beschichtung von medizinischen Implantaten für die medizinische Behandlung von Mensch und Tier.

## Claims

1. Coating material for medical purposes, in particular wound contact material, composed of absorbable synthetic material, **characterized in that** it is formed from a terpolymer based on lactide, trimethylene carbonate and ε-caprolactone with random distribution of the monomers, having a lactide content in the range from 65 to not more than 85 wt. %, a trimethylene carbonate content in the range from 5 to 20 wt. % and an ε-caprolactone content in the range from 5 to 20 wt. %, its degradation behaviour being determined by the fraction ratios of the monomers, in particular the lactide fraction, and the total monomer content in the terpolymer being up to 10 wt. %.

2. Material according to Claim 1, **characterized in that** the monomers lactide/trimethylene carbonate/ε-caprolactone are present in the terpolymer in weight percentage ratios ranging from 85/10/5 to 70/20/10.

3. Material according to Claim 1 or 2, **characterized in that** it has a glass transition point in the temperature range from 20°C to 40°C, particularly in the range from 28 to 37°C.

4. Material according to any one of the preceding claims, **characterized in that** its molecular weight in the sterilized state after treating with gamma rays is in the range from 50 000 to 150 000 Dalton, particularly 60 000 to 90 000 Dalton.

5. Material according to any one of the preceding claims, **characterized in that** its inherent viscosity in the sterilized state after treatment with gamma rays (25 kGy) is in the range from 0.7 to 1.2 dl/g.

6. Material according to any one of the preceding claims, **characterized in that** its elongation is at least 50% at 37°C.

7. Material according to any one of the preceding claims, **characterized in that** its in vivo degradation time is in the range from 15 to 60 days, particularly 20 to 45 days, preferably 25 to 35 days.

8. Material according to any one of the preceding claims, **characterized in that** its in vivo absorption time is in the range from 70 to 120 days, particularly 80 to 100 days.

9. Material according to any one of the preceding claims, **characterized in that** it comprises medically active substances, particularly medicaments.

10. Material according to any one of the preceding claims, **characterized in that** it is a layered material having one or more layers.

11. Material according to any one of the preceding claims, **characterized in that** it is in the form of a self-supporting film.

12. Material according to Claim 10 or Claim 11, **characterized in that** at least one layer is in the form of a porous membrane.

13. Material according to any one of Claims 10 to 12, **characterized in that** at least one layer is in the form of a net.

14. Material according to any one of Claims 10 to 12, **characterized in that** at least one layer is in the form of a hollow membrane.

15. Material according to any one of Claims 10 to 12, **characterized in that** it is in the form of a nonwoven, particularly a melt blown nonwoven.

16. Material according to any one of the preceding claims, **characterized in that** it is porous, its porosity being more than 85% in particular.

17. Material according to any one of the preceding claims, **characterized in that** its pore size is more than 1 µm, particularly more than 10 µm.

18. Material according to any one of the preceding claims, **characterized in that** it is in the form of a solution.

19. Method of manufacturing a coating material for medical purposes, in particular a wound contact material composed of absorbable synthetic material, **characterized in that** a terpolymer based on lactide, trimethylene carbonate and ε-caprolactone, having a lactide content in the range from 65 to not more than 85 wt. %, a trimethylene carbonate content in the range from 5 to 20 wt. % and an ε-caprolactone content in the range from 5 to 20 wt. %, is formed by random polymerization and the terpolymer is formed by polymer-processing methods into a product for medical use, its degradation behaviour being determined by the fraction ratios of the monomers, in particular the lactide fraction, and the total monomer content in the terpolymer being up to 10 wt. %.

20. Method according to Claim 19, **characterized in that** the terpolymer is dissolved in a solvent and formed into sheetlike constructions by spreading techniques.

21. Method according to Claim 19, **characterized in that** the terpolymer is formed thermoplastically by extrusion into filaments or self-supporting films.

22. Method according to Claim 19, **characterized in that** the terpolymer is formed thermoplastically by melt blow technology into porous structures.

23. Method according to Claim 19, **characterized in that** the terpolymer is dissolved in a solvent and formed into a porous structure by phase inversion techniques.

24. Use of a coating material according to any one of Claims 1 to 18 for manufacturing a wound-covering material for the medical treatment of humans and animals.

25. Use of a coating material according to any one of Claims 1 to 18 for manufacturing a skin-replacing material for the medical treatment of humans and animals.

26. Use of a coating material according to any one of Claims 1 to 18 for manufacturing an adhesion prophylaxis material for the medical treatment of humans and animals.

27. Use of a coating material according to any one of Claims 1 to 18 as a matrix for cell cultures.

28. Use of a coating material according to any one of Claims 1 to 18 as a coating for medical implants for the medical treatment of humans and animals.

## Revendications

1. Matériau de revêtement pour la médecine, en particulier matériau de contact avec les blessures, constitué d'un matériau synthétique résorbable, **caractérisé en ce qu'**il est formé d'un terpolymère à base de lactide, de carbonate de triméthylène et d'ε-caprolactone avec une teneur en lactide de 65 à au plus 85 % en poids, une teneur en carbonate de triméthylène comprise dans la plage de 5 à 20 % en poids et une teneur en ε-caprolactone comprise dans la plage de 5 à 20 % en poids, son comportement de décomposition étant défini par les proportions des monomères et en particulier par la teneur en lactide, la teneur totale en monomères dans le terpolymère pouvant atteindre 10 % en poids.

2. Matériau selon la revendication 1, **caractérisé en ce que** dans le terpolymère, les monomères lactide/carbonate de triméthylène/ε-caprolactone sont présents dans des proportions de l'ordre de 85/10/5 à 70/20/10 % en poids.

3. Matériau selon les revendications 1 ou 2, **caractérisé en ce qu'**il présente un point de transition vitreuse dans la plage de 20 à 40°C et en particulier dans la plage de 28 à 37°C.

4. Matériau selon l'une des revendications précédentes, **caractérisé en ce qu'**à l'état stérilisé et après traitement aux rayons gamma, il présente un poids moléculaire de 50 000 à 150 000 Dalton et en particulier de 60 000 à 90 000 Dalton.

5. Matériau selon l'une des revendications précédentes, **caractérisé en ce qu'**à l'état stérilisé et après traitement aux rayons gamma (25 kGy), il présente une viscosité intrinsèque de 0,7 à 1,2 dl/g.

6. Matériau selon l'une des revendications précédentes, **caractérisé en ce qu'**il présente un allongement d'au moins 50 % à 37°C.

7. Matériau selon l'une des revendications précédentes, **caractérisé en ce que** sa durée de dégradation in vivo est de 15 à 60 jours, en particulier de 20 à 45 jours et de préférence de 25 à 35 jours.

8. Matériau selon l'une des revendications précédentes, **caractérisé en ce que** sa durée de résorption in vivo est de 70 à 120 jours et en particulier de 80 à 100 jours.

9. Matériau selon l'une des revendications précédentes, **caractérisé en ce qu'**il contient des substances à action médicale, en particulier des médicaments.

10. Matériau selon l'une des revendications précédentes, **caractérisé en ce qu'**il présente la forme d'un matériau stratifié en une ou plusieurs couches.

11. Matériau selon l'une des revendications précédentes, **caractérisé en ce qu'**il présente la forme d'une feuille.

12. Matériau selon l'une des revendications 10 ou 11, **caractérisé en ce qu'**au moins une couche présente la forme d'une membrane poreuse.

13. Matériau selon l'une des revendications 10 à 12, **caractérisé en ce qu'**au moins une couche présente la forme d'un treillis.

14. Matériau selon l'une des revendications 10 à 12, **caractérisé en ce qu'**au moins une couche présente la forme d'une membrane creuse.

15. Matériau selon l'une des revendications 10 à 12, **caractérisé en ce qu'**il présente la forme d'un non-tissé et en particulier d'un non-tissé obtenu par soufflage à l'état fondu.

16. Matériau selon l'une des revendications précédentes, **caractérisé en ce qu'**il est poreux, sa porosité étant en particulier de plus de 85 %.

17. Matériau selon l'une des revendications précédentes, **caractérisé en ce que** la taille de ses pores est supérieure à 1 µm et en particulier supérieure à 10 µm.

18. Matériau selon l'une des revendications précédentes, **caractérisé en ce qu'**il présente la forme d'une solution.

19. Procédé de fabrication d'un matériau de recouvrement pour la médecine, en particulier un matériau de contact avec les lésions, constitué d'un matériau synthétique résorbable, **caractérisé en ce que** par polymérisation statistique, on forme un terpolymère à base de lactide, de carbonate de triméthylène et d'ε-caprolactone avec une teneur en lactide de 65 à au plus 85 % en poids, une teneur en carbonate de triméthylène comprise dans la plage de 5 à 20 % en poids et une teneur en ε-caprolactone comprise dans la plage de 5 à 20 % en poids, et **en ce que** le polymère est configuré par des procédés de traitement de polymères en un produit destiné à des applications médicales, son comportement de décomposition étant défini par les proportions des monomères et en particulier par la teneur en lactide, la teneur totale en monomères dans le terpolymère pouvant atteindre 10 % en poids.

20. Procédé selon la revendication 19, **caractérisé en ce que** le terpolymère est dissous dans un solvant et est transformé en produits plats par des techniques d'étalement.

21. Procédé selon la revendication 19, **caractérisé en ce que** le terpolymère est réalisé en conditions thermoplastiques par extrusion en filaments ou en feuilles.

22. Procédé selon la revendication 19, **caractérisé en ce que** le terpolymère est transformé en structures poreuses en conditions thermoplastiques par une technique de soufflage à l'état fondu.

23. Procédé selon la revendication 19, **caractérisé en ce que** le terpolymère est dissous dans un solvant et est transformé en une structure poreuse par des techniques d'inversion de phases.

24. Utilisation d'un matériau de revêtement selon l'une des revendications 1 à 18 pour fabriquer un matériau de recouvrement de blessures pour le traitement médical de l'homme ou de l'animal.

25. Utilisation d'un matériau de revêtement selon l'une des revendications 1 à 18 pour préparer un matériau de remplacement de la peau dans le traitement médical de l'homme et de l'animal.

26. Utilisation d'un matériau de revêtement selon l'une des revendications 1 à 18 pour la préparation d'un matériau de prophylaxie de l'adhérence dans le traitement médical de l'homme et de l'animal.

27. Utilisation d'un matériau de revêtement selon l'une des revendications 1 à 18 comme matrices pour des cultures de cellules.

28. Utilisation d'un matériau de revêtement selon l'une des revendications 1 à 18 comme revêtement d'implants médicaux dans le traitement médical de l'homme et de l'animal.
